# FASCICULE DE BREVET EUROPEEN

(11) **EP 1 107 728 B1**
(45) Date de publication et mention de la délivrance du brevet: **28.04.2004**
(21) Numéro de dépôt: 00951616.2
(22) Date de dépôt: 20.06.2000
(51) Int. Cl.: A61K 7/42

(54) **COMPOSITIONS COSMETIQUES PHOTOPROTECTRICES ET UTILISATIONS**
SONNENSCHUTZZUSAMMENSETZUNGEN UND DEREN VERWENDUNG
COSMETIC COMPOSITIONS FOR SOLAR PROTECTION AND USES

(30) Priorité: 02.07.1999 FR 9908568
(43) Date de publication de la demande: 20.06.2001
(73) Titulaire: L'Oreal, 75014 Paris (FR)
(72) Inventeur: AGOSTINI, Isabelle, F-92290 Chatenay Malabry (FR); ARNAUD, Pascal, F-94240 L'Hay les Roses (FR); GUILLARD, Sylvie, F-93140 Bondy (FR)
(74) Mandataire: Miszputen, Laurent
(86) Numéro de dépôt international: PCT/FR2000/001696
(87) Numéro de publication internationale: WO 2001/001946

(56) Documents cités:
- EP-A- 0 711 778
- EP-A- 0 711 779
- EP-A- 0 742 003
- EP-A- 0 893 119
- WO-A-97/37634
- WO-A-99/66896
- FR-A- 2 779 959
- FR-A- 2 783 711

## Description

L'invention concerne des compositions cosmétiques à usage topique, en particulier pour la photoprotection de la peau et/ou des cheveux, caractérisée par le fait qu'elles comprennent, dans un support cosmétiquement acceptable, (a) au moins un dérivé silicié à fonction benzotriazole à titre de premier filtre, et (b) à titre de deuxième filtre, au moins un dérivé de l'acide cinnamique ; lesdits premier et second filtres étant présents dans lesdites compositions dans une proportion produisant une activité synergique au niveau des indices de protection solaire conférés. L'invention concerne également leur application à la protection de la peau et des cheveux contre les effets du rayonnement ultraviolet.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est-à-dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

On connaît dans l'état de la technique des filtres UV cosmétiques des dérivés siliconés à fonction benzotriazole lipophiles présentant de bonnes propriétés filtrantes aussi bien dans le domaine des radiations UV-A que dans le domaine des radiations UV-B. Ils sont décrits dans les demandes EP-A-0392883 ; EP-A-0660701 ; EP-A-0708108 ; EP-A-0711778 ; EP-A-711779.

Dans les demandes EP-A-0742003 et EP-A-0860165, on a déjà proposé d'associer à ces filtres silicones à fonction benzotriazole des filtres hydrosolubles à fonction sulfonique particuliers à savoir l'acide benzène 1,4-di(3-méthylidène-10-camphosulfonique) ou l'acide 2-phényl benzimidazole 5-sulfonique et ses sels, en vue de produire une activité synergique au niveau des indices de protection solaire. Ces systèmes filtrants synergiques imposent l'utilisation d'au moins une phase aqueuse solubilisant le filtre hydrosoluble et d'une phase grasse pour solubiliser le filtre siliconé ; ce qui réduit sensiblement les possibilités de formulation.

Dans la demande EP-A-0835094, on a déjà proposé d'associer à ces filtres silicones à fonction benzotriazole, deux autres filtres lipophiles à savoir un dérivé de dibenzoylméthane et un dérivé de β,β'-diphénylacrylate d'alkyle. La présence obligatoire de ces trois filtres complique là encore leur formulation dans des produits solaires.

On connaît dans la demande de brevet français N° FR 2783711 publiée, des compositions filtrantes à base de dérivés siliciés de benzotriazole à l'état solubilisé contenant au moins un dérivé cinnamique dans une quantité suffisante pour solubiliser à lui seul ledit filtre siliconé. Ce même document décrit en particulier un exemple de formulation sous forme d'émulsion huile-dans-eau contenant 0,5% en poids d'acide stéarique, 2,5% en poids d'alcool stéarylique, 2% en poids de polydiméthylsiloxane, 0,22% de polymère épaississant acrylique, 0,22% en poids de triéthanolamine, 8% en poids d'agent hydratant, 5% en poids de Drométrizole Trisiloxane et 10% en poids de p-méthoxycinnamate de 2-éthylhexyle. Ce document ne se propose pas d'obtenir avec le dérivé silicié de benzotriazole un effet de synergie au niveau des indices de protection.

Or, à la suite d'importantes recherches menées dans le domaine de la photoprotection évoqué ci-dessus, la Demanderesse a découvert, de façon inattendue et surprenante, que la combinaison, dans des proportions comprises dans des limites bien déterminées, d'un filtre du type dérivé silicié de benzotriazole et d'un filtre du type dérivé d'acide cinnamique, produisait un effet de synergie remarquable au niveau des indices de protection. Une telle association permettait d'obtenir des compositions solaires dont les indices de protection solaire sont nettement améliorés, et en tous cas largement supérieurs à ceux qui peuvent être obtenus soit avec l'un ou l'autre des filtres utilisé seul.

De plus, l'association particulière de filtres conforme à l'invention peut être facilement incorporée dans une très large gamme de supports cosmétiques.

Cette découverte est à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable :
(i) à titre de premier filtre, au moins un dérivé silicié à fonction benzotriazole de formule (5) ou (6) telles que définies ci-dessous;
(ii) , à titre de deuxième filtre, au moins un dérivé de l'acide cinnamique choisi parmi le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine ;
   le rapport en poids du dérivé cinnamique sur le dérivé silicié de benzotriazole varie de 0,5/6,5 à 6,75/0,25.
   sous réserve que ladite composition soit différente d'une émulsion huile-dans-eau contenant 2,5% en poids d'acide stéarique, 0,5% en poids d'alcool stéarylique, 2% en poids de polydiméthylsiloxane, 0,22% de polymère épaississant acrylique, 0,22% en poids de triéthanolamine, 8% en poids d'agent hydratant, 5% en poids de Drométrizole Trisiloxane et 10% en poids de p-méthoxycinnamate de 2-éthylhexyle.

La présente invention a également pour objet l'utilisation des compositions ci-dessus pour la fabrication de compositions cosmétiques destinées à la protection de la peau et/ou des cheveux contre le rayonnement ultraviolet, en particulier le rayonnement solaire.

La présente invention a également pour objet l'utilisation d'un dérivé cinnamique pour la fabrication de compositions cosmétiques destinées à la protection de la peau et/ou des cheveux contre le rayonnement ultraviolet, en particulier le rayonnement solaire comprenant au moins un filtre du type dérivé silicié de benzotriazole dans le but de produire un effet synergique au niveau des indices de protection solaire conférés.

D'autres caractéristiques, aspects et avantages de la présente invention apparaîtront à la lecture de la description détaillée qui va suivre.

Les dérivés siliciés à fonction benzotriazole utilisés dans la présente invention sont de préférence des silanes ou des siloxanes à fonction benzotriazole comprenant au moins une unité de formule (1) suivante :

O_{(3-a)/2} Si (R₇)ₐ - G (1)

dans laquelle :
- R₇ représente un radical alkyle en C₁-C₁₀ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante : 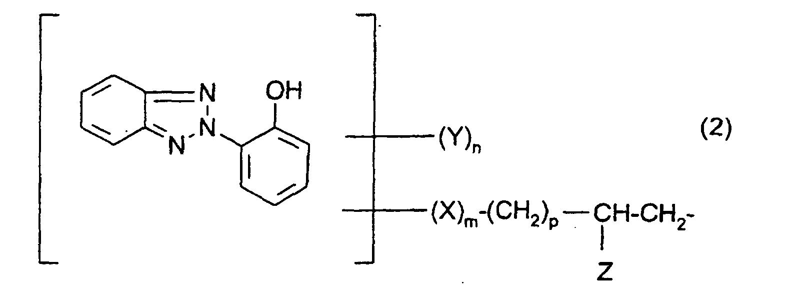
dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.

De préférence, les dérivés siliciés utilisés dans le cadre de la présente invention appartiennent à la famille générale des silicones benzotriazoles qui est décrite notamment dans EP-A-0660701 .

Une famille de silicones benzotriazoles convenant particulièrement bien à la réalisation de la présente invention est celle regroupant les composés répondant aux formules (5) ou (6) suivantes : 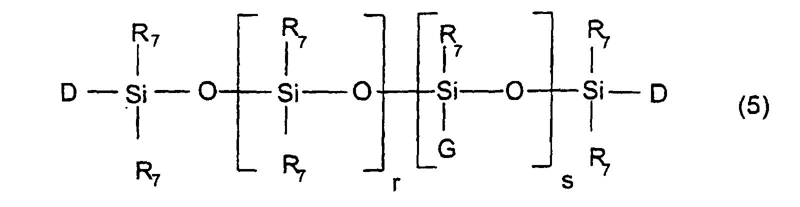 ou 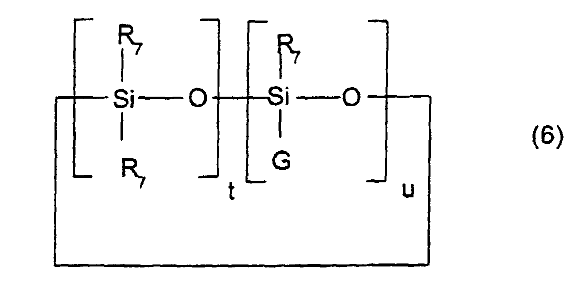 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

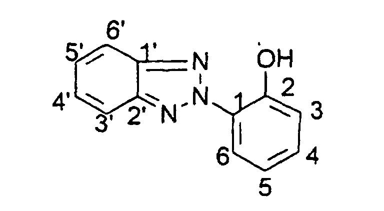

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires
ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R₇ préférés selon l'invention sont les radicaux méthyle, éthyle, propyle, n-butyle, n-octyle et éthyl-2 hexyle. Encore plus préférentiellement, les radicaux R₇ sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formules (5) ci-dessus, on préfère mettre en oeuvre ceux pour lesquels les radicaux D sont tous les deux des radicaux R₇

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- D est un radical R₇
- R₇ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en C₁-C₄,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante : 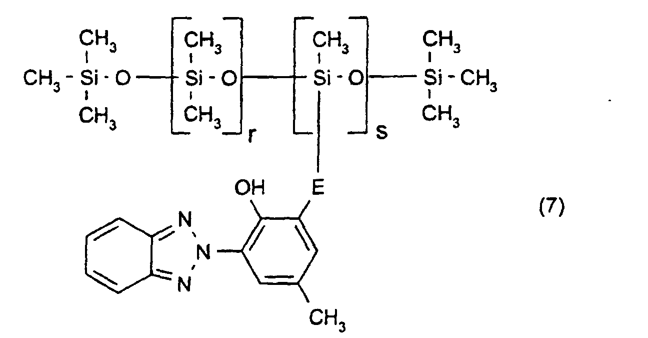 avec

0 ≤ r ≤ 10 ,

1 ≤ s ≤ 10 ,

et où E représente le radical divalent :

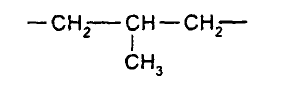

Dans une forme particulièrement préférée de réalisation de l'invention, la silicone benzotriazole est le composé Drométrizole Trisiloxane (nom CTFA) répondant à la formule suivante :

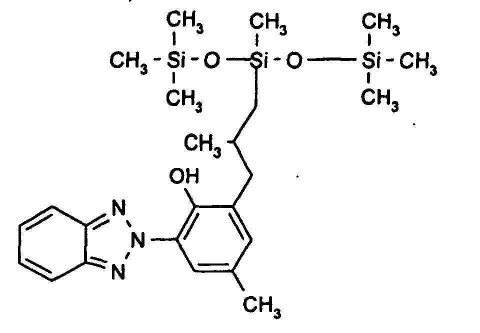

Des procédés convenant à la préparation des produits de formule (1), (5), (6) et (7) ci-dessus sont notamment décrits dans les brevets américains US3,220,972, US3,697,473, US4,340,709, US4,316,033, US4,328,346 et dans les demandes de brevet EP-A-0392883 et EP-A-0742 003.

Le dérivé silicié à fonction benzotriazole peut être présent dans les compositions selon l'invention à des teneurs allant de 0,1 à 20%, de préférence allant de 0,2 à15%, en poids, toujours par rapport au poids total de la composition.

Parmi les dérivés de l'acide cinnamique mentionnés ci-dessus, on préfère tout particulièrement, selon la présente invention, mettre en oeuvre le p-méthoxycinnamate de 2-éthylhexyle, vendu notamment sous le nom commercial « PARSOL MCX » par la Société GIVAUDAN ; ce filtre répondant donc à la formule développée suivante :

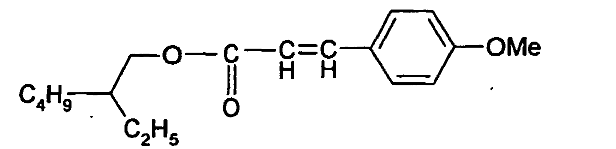

Le ou les dérivés de l'acide cinnamique de l'invention sont présents à des teneurs allant de 0,1 à 20 % en poids et de préférence allant de 0,2 à 15% par rapport au poids total de la composition.

Comme indiqué précédemment, selon une caractéristique fonctionnelle essentielle de la présente invention, il convient que les deux types de filtres solaires soient tous deux présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en composés dérivé silicié de benzotriazole et en composés dérivés d'acide cinnamique tels que définis précédemment sont choisis de manière telle que l'indice de protection solaire de la composition finale soit de préférence d'au moins 2.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles; autres bien sûr que les deux filtres mentionnés ci-avant. Ces filtres complémentaires peuvent être notamment choisis parmi les dérivés salicyliques, les dérivés du camphre ; les dérivés de triazine tels que ceux décrits dans les demandes de brevet EP863145, EP517104, EP570838, EP796851, EP775698 et EP878469 ; les dérivés de la benzophénone; les dérivés du dibenzoylméthane ; les dérivés de β,β'-diphénylacrylate, les dérivés de benzimidazole ; les dérivés de l'acide p-aminobenzoïque ; les dérivés hydrocarbonés de bis-(benzotriazolyl-phénol) tels que ceux décrits dans les demandes GB-A-2303549, DE 19726184 et EP-A-893119 ; les composés comportant au moins deux groupes benzoazolyle tels que décrits dans la demande EP-A-0669323 ; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
l'acide benzène 1,4-di(3-méthylidène-10-camphosulfonique) et ses sels,
l'acide urocanique,
la 2,4,6-tris-[ p-(2'-éthylhexyl-1'-oxycarbonyl)anilino]-1,3,5-triazine,
2-[(p-(tertiobutylamido)anilino]-4,6-bis-[(p-(2'-éthylhexyl-1'-oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl]benzyl]-acrylamide,
l'acide 4,4-bis-benzimidazolyl-phénylèn-3,3',5,5'-tétrasulfonique et ses sels
le 2,2'-méthylèn-bis-[6-(2H-benzotriazol-2-yl)-4-(1,1,3,3-tétraméthylbutyl)phénol].
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile, ou encore des compositions anhydres.

Cette composition peut se présenter en particulier sous forme d'émulsion, simple ou complexe (H/E, E/H, H/E/H ou E/H/E) telle qu'une crème, un lait, un gel ou un gel crème, de poudre, de composition solide, de pâtes souples et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de stick, de pâtes souples, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Une forme particulière de l'invention est un stick pour le soin et/ou le maquillage des lèvres comprenant au moins l'une des compositions telles que définies précédemment.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLES 1 à 7:

| **Support commun : stick de soin pour les lèvres** | **% en poids** |
|---|---|
| Cire microcristalline | 5,00 |
| Trihydroxystéarate de glycéryle | 5,00 |
| Ozokérite | 3,40 |
| Cire d'abeille polyglycérolée | 2,10 |
| Lanoline acétylée | 19,45 |
| Huile de lanoline | 19,10 |
| Huile d'avocat | 18,99 |
| Copolymère butène/isobutène | 14,34 |
| Huile de Ricin | 4,81 |
| Palmitate d'ascorbyle | 0,50 |
| Mélange de tocophérols dans huile de soja (50/50) | 0,31 |
| Para-méthoxycinnamate d'éthyle 2-hexyle (PARSOL MCX) | X |
| (*) Drométrizole Trisiloxane | Y |
| Avec X+ Y = 7% en poids | |

Le Drométrizole Trisiloxane a comme structure :

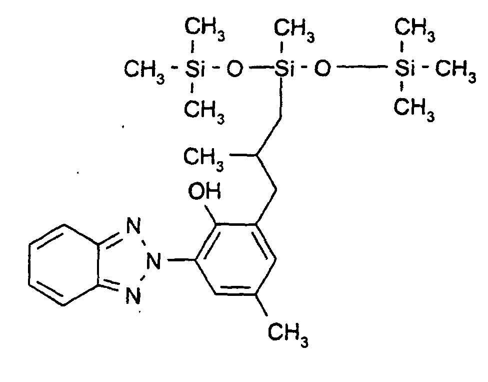

Pour chacun des exemples 1 à 7 ci-dessous, on a déterminé le facteur de protection solaire (FPS) qui lui était attaché. Celui-ci a été déterminé en utilisant la méthode *in vitro* décrite par B.L. DIFFEY et al. dans J. Soc. Cosmet. Chem. 40, 127-133,(1989). Les mesures ont été réalisées à l'aide d'un spectrophotomètre UV-visible modèle SPF 290 S de chez Optometrics muni d'une sphère d'intégration et d'une lampe Xénon.

Chaque exemple de formulation cosmétique est appliqué sur un ruban adhésif TRANSPORE de chez 3M collé sur une lame de quarz , sous la forme d'un dépôt homogène et régulier à raison de 2 mg/cm².

Les compositions des différentes formulations étudiées et les résultats en facteur de protection solaire moyen (moyenne sur cinq essais) obtenus sont rassemblés dans le tableau donné ci-après.

| **Filtre** | **Ex1** | **Ex2** | **Ex3** | **Ex4** | **Ex5** | **Ex6** | **Ex7** | **Ex8** | **Ex9** | **EX 10** | **Ex 11** | **Ex 12** |
|---|---|---|---|---|---|---|---|---|---|---|---|---|
| **X%** | 7 | 6,75 | 6,50 | 6,00 | 5,00 | 4,00 | 3,00 | 2,00 | 1,00 | 0,50 | 0,25 | 0 |
| **Y%** | 0 | 0,25 | 0,50 | 1,00 | 2,00 | 3,00 | 4,00 | 5,00 | 6,00 | 6,50 | 6,75 | 7 |
| **SPF moyen ± écart type** | 9,8 ± 0,3 | 10,6 ± 0,7 | 13,0 ± 0,5 | 13,4 ± 0,7 | 12,3 ± 2,1 | 12,4 ± 2,1 | 12,5 ± 1,8 | 11,4 ± 1,4 | 12,5 ± 2,0 | 11,8 ± 1,7 | 13,0 ± 1,7 | 8,9 ± 0,9 |

Les résultats montrent que l'on obtient un effet de synergie significatif en combinant le dérivé cinnamique et le dérivé silicié de benzotriazole dans des rapports en poids dérivé cinnamique sur dérivé silicié de benzotriazole allant de 0,50/6,50 à 6,75/0,25.

## Revendications

1. Composition cosmétique à usage topique, en particulier pour la photoprotection de la peau et/ou des cheveux, **caractérisées par le fait qu'**elles comprennent, dans un support cosmétiquement acceptable :
(i) à titre de premier filtre, au moins un dérivé silicié à fonction benzotriazole (A) répondant à l'une des formules (5) ou (6) suivantes : 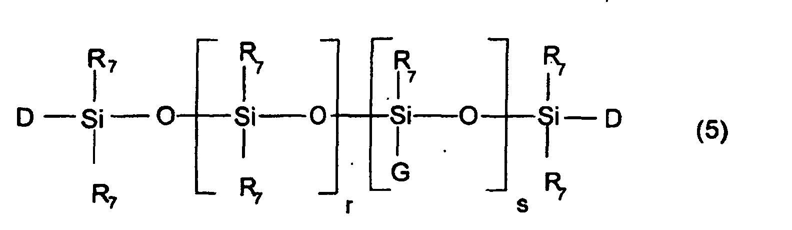 ou 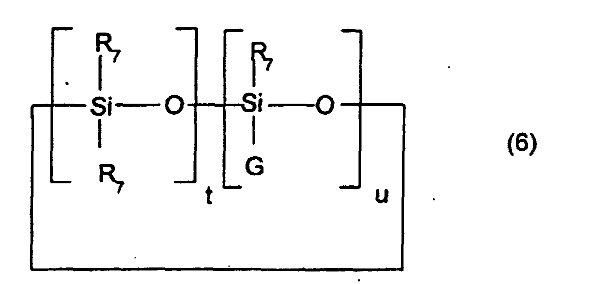 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) suivante : 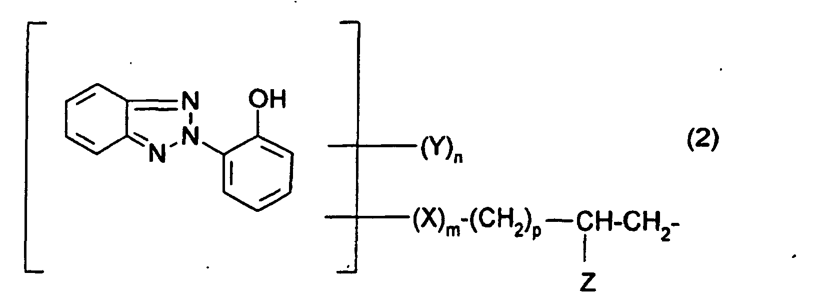
dans laquelle:
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement;
(ii) à titre de deuxième filtre, au moins un dérivé de l'acide cinnamique (B) choisi dans le groupe constitué par le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine ;
le rapport en poids du dérivé cinnamique sur le dérivé silicié de benzotriazole variant de 0,5/6,5 à 6,75/0,25 ;
sous réserve que ladite composition soit différente d'une émulsion huile-dans-eau contenant 2,5% en poids d'acide stéarique, 0,5% en poids d'alcool stéarylique, 2% en poids de polydiméthylsiloxane, 0,22% de polymère épaississant acrylique, 0,22% en poids de triéthanolamine, 8% en poids d'agent hydratant, 5% en poids de Drométrizole Trisiloxane et 10% en poids de p-méthoxycinnamate de 2-éthylhexyle.

2. Composition selon la revendication 1, **caractérisée par le fait que** dérivé silicié à fonction benzotriazole répond à la formule (7) suivante : 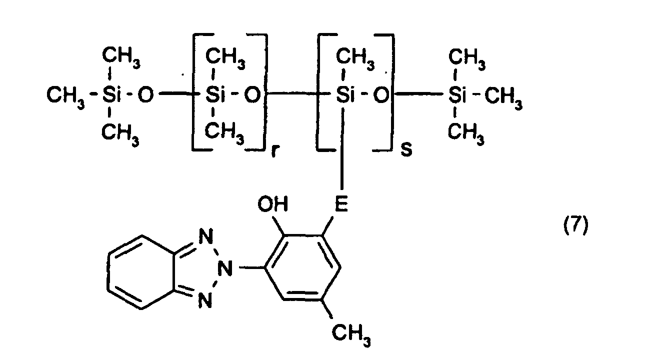 avec
0 ≤ r ≤ 10,
1 ≤ s ≤ 10,
et où E représente le radical divalent :

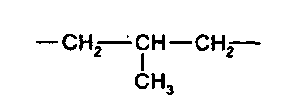

3. Composition selon la revendication 2, **caractérisée par le fait que** le dérivé silicié à fonction benzotriazole est le Drométrizole Trisiloxane de formule suivante :

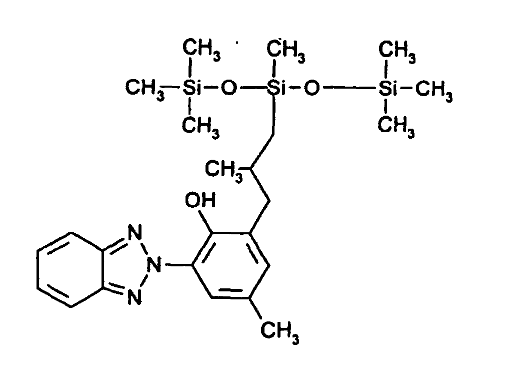

4. Composition selon l'une quelconque des revendications 1 à 3, **caractérisée par le fait que** les dérivés siliciés à fonction benzotriazole sont présents à une teneur allant de 0,1% à 20 %, de préférence de 0,2 % à 15 % en poids, par rapport au poids total de la composition.

5. Composition selon l'une quelconque des revendications 1 à 4, où le dérivé de l'acide cinnamique est le p-méthoxycinnamate de 2-éthylhexyle répondant à la formule développée suivante :

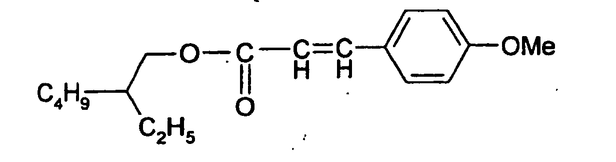

6. Composition selon l'une quelconque des revendications 1 à 5, où le ou dérivés de l'acide cinnamique sont présents à des teneurs allant de 0,1 à 20 % en poids et de préférence allant de 0,2 à 15% par rapport au poids total de la composition.

7. Composition selon l'une quelconque des revendications 1 à 6, **caractérisée par le fait qu'**elles comprend en outre un ou plusieurs filtres organiques complémentaires actifs dans l'UV-A et/ou UV-B, hydrophiles ou lipophiles.

8. Composition selon la revendication 7, **caractérisée par le fait que** lesdits filtres organiques complémentaires sont choisis parmi les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les dérivés bis-(benzotriazolyl-phénol), les composés comportant au moins deux groupements benzoazolyle, les polymères filtres et les silicones filtres autres que les dérivés siliciés à fonction benzotriazole.

9. Composition selon l'une quelconque des revendications 1 à 8, **caractérisée par le fait qu'**elle comprend en outre, à titre d'agents photoprotecteurs complémentaires, des pigments ou nanopigments d'oxydes métalliques, enrobés
ou non, capables de bloquer physiquement, par diffusion et/ou réflection, le rayonnement UV.

10. Compositions selon la revendication 9, **caractérisée par le fait que** lesdits pigments ou nanopigments sont choisis parmi les oxydes de titane, de zinc, de fer, de zirconium, de cérium et leurs mélanges, enrobés ou non.

11. Composition selon l'une quelconque des revendications 1 à 10, **caractérisée par le fait qu'**elles comprend en outre au moins un agent de bronzage et/ou de brunissage artificiel de la peau.

12. Composition selon l'une quelconque des revendications 1 à 11, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

13. Composition selon l'une quelconque des revendications 1 à 12, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

14. Composition selon l'une quelconque des revendications 1 à 13, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

15. Composition selon l'une quelconque des revendications 1 à 14, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

16. Stick pour le soin et/ou le maquillage des lèvres **caractérisé par le fait qu'**elle comprend au moins une composition telle que définie selon l'une quelconque des revendications 1 à 14.

17. Utilisation de la composition définie à l'une quelconque des revendications précédentes pour la fabrication de produits cosmétiques pour la protection de la peau et/ou des cheveux contre le rayonnement ultraviolet, en particulier le rayonnement solaire.

18. Utilisation d'au moins un dérivé de l'acide cinnamique tel que défini dans la revendication 1 pour la fabrication de compositions cosmétiques pour la protection de la peau et/ou des cheveux contre le rayonnement ultraviolet, en particulier le rayonnement solaire comprenant comme filtre UV au moins un dérivé silicié de benzotriazole tel que défini dans l'une quelconque des revendications 1 à 3 dans le but de produire un effet de synergie au niveau des indices de protection solaire conférés.

## Patentansprüche

1. Kosmetische Zusammensetzung zur topischen Anwendung, insbesondere zum Lichtschutz der Haut und/oder der Haare, **dadurch gekennzeichnet, dass** sie in einem kosmetisch akzeptablen Träger enthält:
(i) als erstes Filter mindestens ein siliciumhaltiges Derivat mit Benzotriazolgruppe (A), das einer der folgenden Formeln (5) oder (6) entspricht: 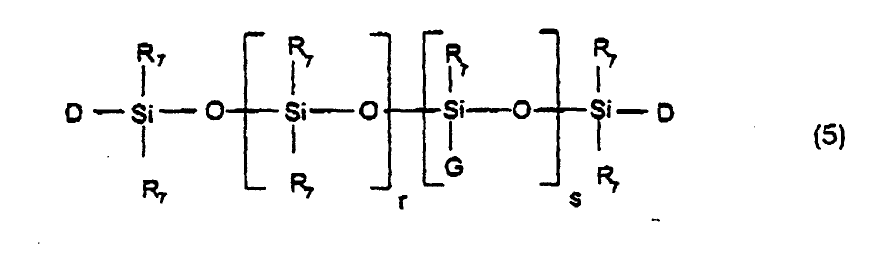 oder 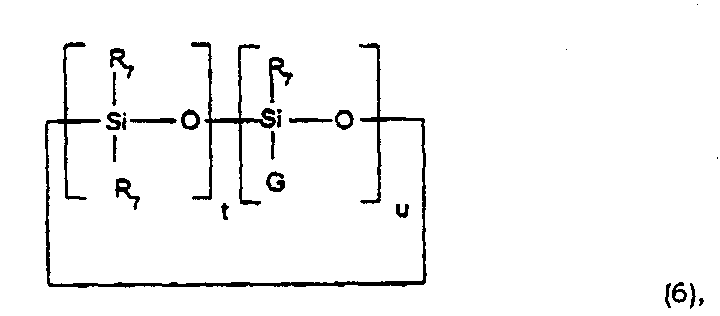 worin:
- die Gruppen R₇, die identisch oder voneinander verschieden sind, unter den C₁₋₁₀-Alkylgruppen, Phenyl, 3,3,3-Trifluorpropyl und Trimethylsilyloxy ausgewählt sind, wobei mindestens 80 % der Anzahl der Gruppen R₇ Methyl bedeutet,
- die Gruppen D, die identisch oder voneinander verschieden sind, unter den Gruppen R₇ und der Gruppe G ausgewählt sind,
- r 0 oder eine ganze Zahl im Bereich von 1 bis 50 und s 0 oder eine ganze Zahl im Bereich von 1 bis 20 bedeutet,
wobei die Bereichsgrenzen jeweils eingeschlossen sind und mindestens eine der beiden Gruppen D G bedeutet, wenn s 0 ist,
- u eine ganze Zahl im Bereich von 1 bis 6 und t 0 oder eine ganze Zahl im Bereich von 1 bis 10 ist, wobei die Bereichsgrenzen jeweils eingeschlossen sind, mit der Maßgabe, dass t + u 3 bedeutet oder darüber liegt, und die Gruppe G der folgenden Formel (2) entspricht: 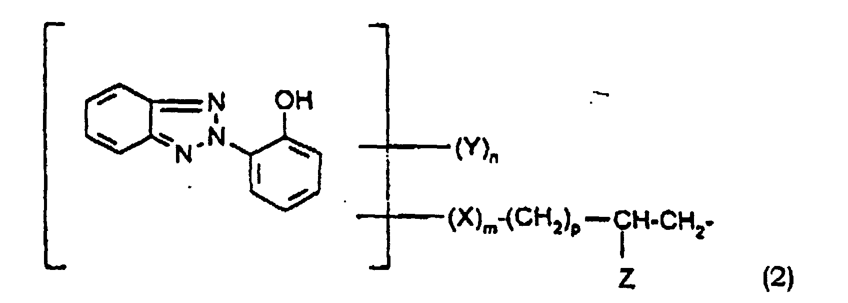
worin:
- die Gruppen Y, die identisch oder voneinander verschieden sind, unter den C₁₋₈-Alkylgruppen, Halogenen und C₁₋₄-Alkoxygruppen ausgewählt sind, wobei im letzten Fall zwei an dem gleichen aromatischen Ring aneinander angrenzende Gruppen Y auch gemeinsam eine Alkylidendioxygruppe bilden können, worin die Alkylidengruppe 1 bis 2 Kohlenstoffatome aufweist,
- X O oder NH bedeutet,
- Z Wasserstoff oder eine C₁₋₄-Alkylgruppe bedeutet,
- n 0 oder eine ganze Zahl im Bereich von 1 bis 3 ist, wobei die Bereichsgrenzen eingeschlossen sind,
- m 0 oder 1 ist, und
- p eine ganze Zahl im Bereich von 1 bis 10 bedeutet, wobei die Bereichsgrenzen eingeschlossen sind; und
(ii) als zweites Filter mindestens eine Zimtsäurederivat (B), das unter Isopentyl-4-methoxy-cinnamat, 2-Ethylhexyl-4-methoxy-cinnamat, Methyl-diisopropyl-cinnamat, Isoamyl-4-methoxy-cinnamat und Diethanolamin-4-methoxy-cinnamat ausgewählt ist,
wobei das Gewichtsverhältnis von Zimtsäurederivat und dem siliciumhaltigen Derivat mit Benzotriazolgruppe im Bereich von 0,5/6,5 bis 6,75/0,25 liegt,
mit der Maßgabe, dass die Zusammensetzung von einer Öl-in-Wasser-Emulsion verschieden ist, die 2,5 Gew.-% Stearinsäure, 0,5 Gew.-% Stearylalkohol, 2 Gew.-% Polydimethylsiloxan, 0,22 % verdickendes Acrylpolymer, 0,22 Gew.-% Triethanolamin, 8 Gew.-% Hydratisierungsmittel, 5 Gew.-% Drometrizole Trisiloxane und 10 Gew.-% 2-Ethylhexyl-p-methoxycinnamat enthält.

2. Zusammensetzung nach Anspruch 1, **dadurch gekennzeichnet, dass** das siliciumhaltige Derivat mit Benzotriazolgruppe der folgenden Formel (7) entspricht: 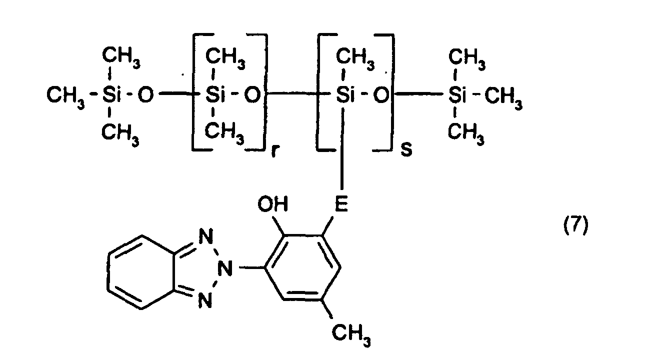 worin bedeuten:
0 ≤ r ≤ 10,
1 ≤ s ≤ 10,
und E die zweiwertige Gruppe:

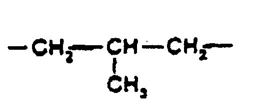

3. Zusammensetzung nach Anspruch 2, **dadurch gekennzeichnet, dass** das siliciumhaltige Derivat mit Benzotriazolgruppe das Drometrizole Trisiloxane der folgenden Formel ist:

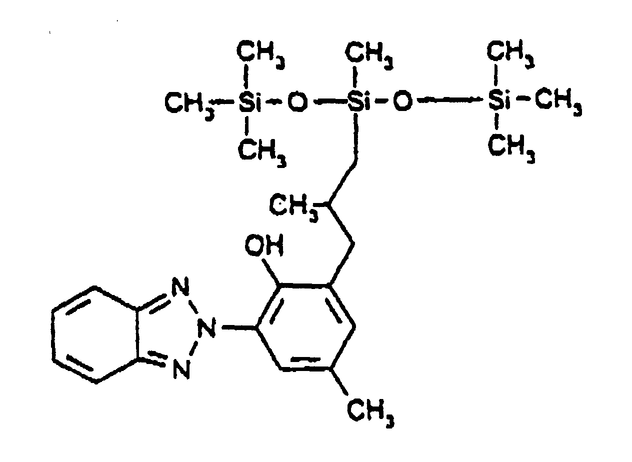

4. Zusammensetzung nach einem der Ansprüche 1 bis 3, **dadurch gekennzeichnet, dass** das siliciumhaltige Derivat mit Benzotriazolgruppe in einem Mengenanteil von 0,1 bis 20 Gew.-% und vorzugsweise 0,2 bis 15 Gew.-%, bezogen auf das Gesamtgewicht der Zusammensetzung, enthalten ist.

5. Zusammensetzung nach einem der Ansprüche 1 bis 4, wobei das Zimtsäurederivat das 2-Ethylhexyl-p-methoxycinnamat der folgenden Strukturformel ist:

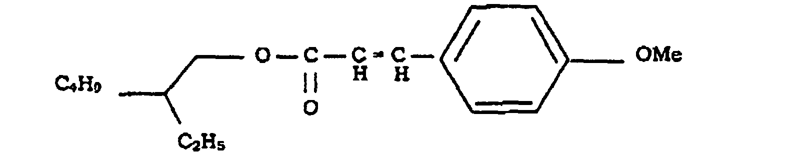

6. Zusammensetzung nach einem der Ansprüche 1 bis 5, wobei das oder die Zimtsäurederivat(e) in einer Menge von 0,1 bis 20 Gew.-% und vorzugsweise 0,2 bis 15 %, bezogen auf das Gesamtgewicht der Zusammensetzung, enthalten sind.

7. Zusammensetzung nach einem der Ansprüche 1 bis 6, **dadurch gekennzeichnet, dass** sie ferner ein oder mehrere, ergänzende, hydrophile oder lipophile, organische Filter, die im UV-A-Bereich und/oder UV-B-Bereich wirksam sind, enthält.

8. Zusammensetzung nach Anspruch 7, **dadurch gekennzeichnet, dass** die ergänzenden organischen Filter unter den Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, Bis(benzotriazolyl-phenol)-derivaten, Verbindungen, die mindestens zwei Benzoazolylgruppen enthalten, polymeren Filtern und Siliconfiltern, die von den beschriebenen siliciumhaltigen Derivaten mit Benzotriazolfunktion verschieden sind, ausgewählt werden.

9. Zusammensetzung nach einem der Ansprüche 1 bis 8, **dadurch gekennzeichnet, dass** sie als ergänzende Lichtschutzmittel ferner gegebenenfalls umhüllte Pigmente oder Nanopigmente von Metalloxiden enthält, die die UV-Strahlung physikalisch durch Streuung und/ oder Reflexion zu sperren vermögen.

10. Zusammensetzung nach Anspruch 9, **dadurch gekennzeichnet, dass** die Pigmente oder Nanopigmente unter den gegebenenfalls umhüllten Oxiden von Titan, Zink, Eisen, Zirconium, Cer und deren Gemischen ausgewählt sind.

11. Zusammensetzung nach einem der Ansprüche 1 bis 10, **dadurch gekennzeichnet, dass** sie außerdem mindestens ein Bräunungsmittel und/oder Mittel zur künstlichen Braunfärbung der Haut enthält.

12. Zusammensetzung nach einem der Ansprüche 1 bis 11, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Antioxidantien, Trübungsmitteln, Stabilisatoren, Emollientien, Hydroxysäuren, Schaumverhütungsmitteln, Hydratisierungsmitteln, Vitaminen, Parfums, Konservierungsmitteln, grenzflächenaktiven Stoffen, Füllstoffen, Maskierungsmitteln, Polymeren, Treibmitteln, Alkalisierungsmitteln, Ansäuerungsmitteln und Farbmitteln ausgewählt ist.

13. Zusammensetzung nach einem der Ansprüche 1 bis 12, **dadurch gekennzeichnet, dass** es sich um Zusammensetzungen, die die menschliche Epidermis schützen, oder Sonnenschutzzusammensetzungen handelt und diese als nichtionische Vesikeldispersionen, Emulsionen, insbesondere Öl-in-Wasser-Emulsionen, Cremes, Milche, Gele, Gelcremes, Suspensionen, Dispersionen, Puder, feste Stifte, Schäume und Sprays vorliegen.

14. Zusammensetzung nach einem der Ansprüche 1 bis 13, **dadurch gekennzeichnet, dass** es sich um Zusammensetzungen zum Schminken der Wimpern, Augenbrauen oder der Haut handelt und dass sie in fester oder pastöser, wasserfreier oder wässriger Form, als Emulsionen, Suspensionen oder Dispersionen vorliegen.

15. Zusammensetzung nach einem der Ansprüche 1 bis 14, **dadurch gekennzeichnet, dass** es sich um Zusammensetzungen zum Schutz der Haare gegen UV-Strahlung handelt und dass sie als Haarwaschmittel, Lotionen, Gele, Emulsionen oder nichtionische Vesikeldispersionen vorliegen.

16. Stift zur Pflege und/oder zum Schminken der Lippen, **dadurch gekennzeichnet, dass** er mindestens eine Zusammensetzung nach einem der Ansprüche 1 bis 14 enthält.

17. Verwendung der Zusammensetzung nach einem der vorhergehenden Ansprüche für die Herstellung von kosmetischen Produkten zum Schutz der Haut und/oder der Haare gegen UV-Strahlung und insbesondere gegen Sonnenlicht.

18. Verwendung mindestens eines in Anspruch 1 definierten Zimtsäurederivats für die Herstellung von kosmetischen Zusammensetzungen, die für den Schutz der Haut und/oder der Haare gegen UV-Strahlung und insbesondere gegen Sonnenlicht vorgesehen sind und die als UV-Filter mindestens ein siliciumhaltiges Derivat mit Benzotriazolgruppe enthalten, um hinsichtlich der Lichtschutzfaktoren eine synergistische Wirkung zu erzielen.

## Claims

1. Cosmetic composition for topical use, in particular for sunblock protection of the skin and/or the hair, **characterized in that** it comprises, in a cosmetically acceptable support:
(i) as first screening agent, at least one silicon derivative containing a benzotriazole function (A) corresponding to either of the formulae (5) and (6) below: 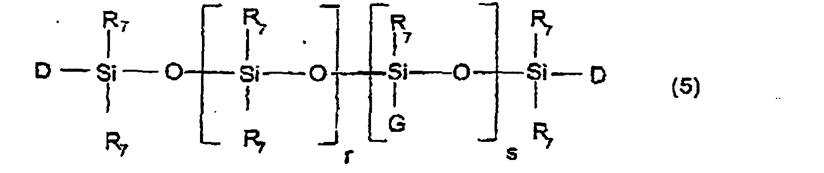 and 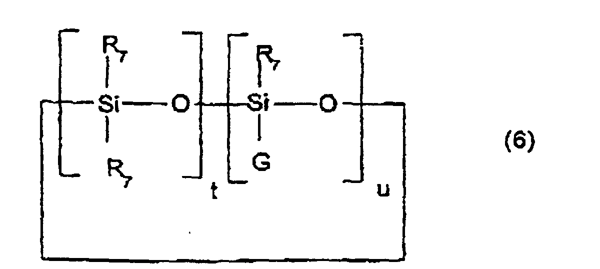 in which:
- R₇, which may be identical or different, are chosen from C₁-C₁₀ alkyl, phenyl, 3,3,3-trifluoropropyl and trimethylsilyloxy radicals, at least 80%, on a number basis, of the radicals R₇ being methyl,
- D, which may be identical or different, are chosen from the radicals R₇ and the radical G,
- r is an integer between 0 and 50 inclusive and s is an integer between 0 and 20 inclusive, and if s = 0, at least one of the two symbols D denotes G,
- u is an integer between 1 and 6 inclusive and t is an integer between 0 and 10 inclusive, it being understood that t + u is equal to or greater than 3,
- and the symbol G corresponds to formula (2) below: 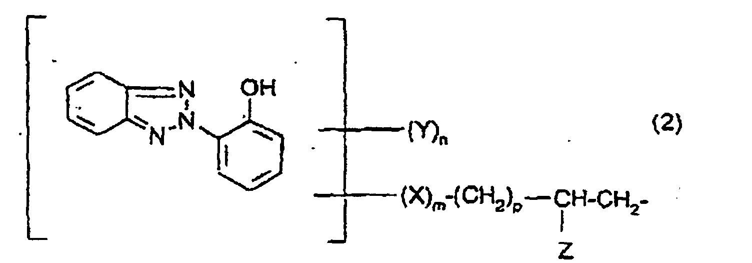
in which:
- Y, which may be identical or different, are chosen from C₁-C₈ alkyl radicals, halogens and C₁-C₄ alkoxy radicals, it being understood that, in the latter case, two adjacent groups Y on the same aromatic ring can together form an alkylidenedioxy group in which the alkylidene group contains 1 or 2 carbon atoms,
- X represents O or NH,
- Z represents hydrogen or a C₁-C₄ alkyl radical,
- n is an integer between 0 and 3 inclusive,
- m is 0 or 1,
- p represents an integer between 1 and 10 inclusive;
(ii) as second screening agent, at least one cinnamic acid derivative (B) chosen from the group consisting of isopentyl 4-methoxycinnamate, 2-ethylhexyl 4-methoxycinnamate, methyl diisopropylcinnamate, isoamyl 4-methoxycinnamate and diethanolamine 4-methoxycinnamate;
the weight ratio of the cinnamic derivative to the benzotriazole silicon derivative ranging from 0.5/6.5 to 6.75/0.25;
with the proviso that the said composition is other than an oil-in-water emulsion containing 2.5% by weight of stearic acid, 0.5% by weight of stearyl alcohol, 2% by weight of polydimethylsiloxane, 0.22% of acrylic thickening polymer, 0.22% by weight of triethanolamine, 8% by weight of moisturizer, 5% by weight of drometrizole trisiloxane and 10% by weight of 2-ethylhexyl p-methoxycinnamate.

2. Composition according to Claim 1, **characterized in that** the silicon derivative containing a benzotriazole function corresponds to formula (7) below: 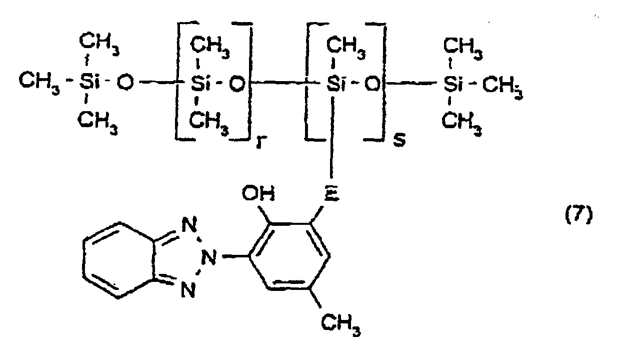 with
0 ≤ r ≤ 10,
1 ≤ s ≤ 10,
and where E represents the divalent radical:

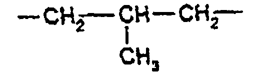

3. Composition according to Claim 2, **characterized in that** the silicon derivative containing a benzotriazole function is drometrizole trisiloxane, of the following formula:

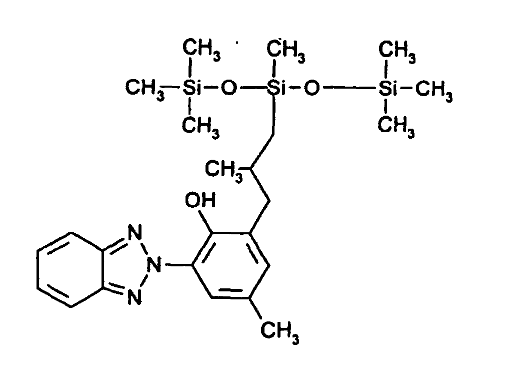

4. Composition according to any one of Claims 1 to 3, **characterized in that** the silicon derivatives containing a benzotriazole function are present in a content ranging from 0.1% to 20% by weight, preferably from 0.2% to 15% by weight, relative to the total weight of the composition.

5. Composition according to any one of Claims 1 to 4, in which the cinnamic acid derivative is 2-ethylhexyl p-methoxycinnamate corresponding to the following structural formula:

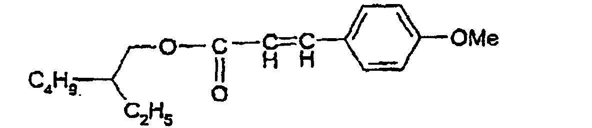

6. Composition according to any one of Claims 1 to 5, in which the cinnamic acid derivative(s) is (are) present at contents ranging from 0.1% to 20% by weight and preferably ranging from 0.2% to 15% by weight relative to the total weight of the composition.

7. Composition according to any one of Claims 1 to 6, **characterized in that** it also comprises one or more additional hydrophilic or lipophilic organic screening agents that are active in the UV-A and/or UV-B range.

8. Composition according to Claim 7, **characterized in that** the said additional organic screening agents are chosen from salicylic derivatives, camphor derivatives, triazine derivatives, benzophenone derivatives, dibenzoylmethane derivatives, β,β-diphenylacrylate derivatives, p-aminobenzoic acid derivatives, bis(benzotriaaolylphenol) derivatives, compounds comprising at least two benzazolyl groups, and screening polymers and screening silicones other than the silicon derivatives containing a benzotriazole function.

9. Composition according to any one of Claims 1 to 8, **characterized in that** it also comprises, as additional sunblock agents, coated or uncoated pigments or nanopigments of metal oxides, capable of physically blocking out UV radiation, by scattering and/or reflection.

10. Compositions according to Claim 9, **characterized in that** the said pigments or nanopigments are chosen from titanium oxide, zinc oxide, iron oxide, zirconium oxide and cerium oxide and mixtures thereof, which are coated or uncoated.

11. Composition according to any one of Claims 1 to 10, **characterized in that** it also comprises at least one agent for artificially tanning and/or browning the skin.

12. Composition according to any one of Claims 1 to 11, **characterized in that** it also comprises at least one adjuvant chosen from fatty substances, organic solvents, thickeners, softeners, antioxidants, opacifiers, stabilizers, emollients, hydroxy acids, antifoaming agents, moisturizers, vitamins, fragrances, preserving agents, surfactants, fillers, sequestering agents, polymers, propellants, acidifying or basifying agents, and dyes.

13. Composition according to any one of Claims 1 to 12, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream-gel, a suspension, a dispersion, a powder, a solid stick, a foam or a spray.

14. Composition according to any one of Claims 1 to 13, **characterized in that** it is a make-up composition for the eyelashes, the eyebrows or the skin and **in that** it is in solid or pasty, anhydrous or aqueous form, or in the form of an emulsion, a suspension or a dispersion.

15. Composition according to any one of Claims 1 to 14, **characterized in that** it is a composition intended for protecting the hair against ultraviolet rays and **in that** it is in the form of a shampoo, a lotion, a gel, an emulsion or a nonionic vesicular dispersion.

16. Care and/or make-up stick for the lips, **characterized in that** it comprises at least one composition as defined in any one of Claims 1 to 14.

17. Use of the composition defined in any one of the preceding claims, to manufacture cosmetic products for protecting the skin and/or the hair against ultraviolet radiation, in particular solar radiation.

18. Use of at least one cinnamic acid derivative as defined in Claim 1, to manufacture cosmetic compositions for protecting the skin and/or the hair against ultraviolet radiation, in particular solar radiation, comprising, as UV screening agent, at least one benzotriazole silicon derivative as defined in any one of Claims 1 to 3, with the aim of producing a synergistic effect on the sun protection factors imparted.
